# EUROPEAN PATENT APPLICATION

(11) **EP 1 266 655 A1**
(43) Date of publication of application: **18.12.2002**
(21) Application number: 01912475.9
(22) Date of filing: 19.03.2001
(51) Int. Cl.: A61K 9/16, A61K 47/10, A61K 47/12, A61K 47/44, A61K 47/38, A61K 47/32, A61K 9/48

(54) **ENTERIC GRANULAR PREPARATIONS OF HARDLY WATER SOLUBLE DRUGS CHARACTERIZED BY CONTAINING WATER-REPELLENT COMPONENT**

(30) Priority: 23.03.2000 JP 2000081354
(71) Applicant: SHIONOGI & CO., LTD., Osaka-shi, Osaka 541-0045 (JP)
(72) Inventor: TSUKADA, Takayuki, c/o Shionogi & Co., Ltd., Amagasaki-shi,Hyogo 660-0813 (JP); SYODAI, Hidekazu, c/o Shionogi & Co., Ltd., Amagasaki-shi, Hyogo 660-0813 (JP); SEZAKI, Hajime, c/o Shionogi & Co., Ltd., Amagasaki-shi, Hyogo 660-0813 (JP); SUZUKI, Yusuke, c/o Shionogi & Co., Ltd., Settsu-shi, Osaka 566-0022 (JP)
(74) Representative: Baverstock, Michael George Douglas
(86) International application number: JP0102152
(87) International publication number: WO01070201

(57) **Abstract**

A burst caused by an action of digestive organs can be suppressed and a release of an active ingredient can be controlled without decreasing the dissolution of an active ingredient by adding a water repellent agent into an enteric coat, an inner layer, an outer layer or such layers.

## Description

### Technical Field

The present invention relates to an enteric coat granule, in detail, an enteric coat granule wherein a burst caused by an action of digestive organs is suppressed, and in more detail, an enteric coat granule comprising a water repellent agent.

### Background Art

Generally, when an active ingredient is insoluble, an insoluble coating type or matrix type of sustained release formulation extremely decreases the eluting rate to cause the reduction of the absorption. Therefore, the matrix type or reservoir type of formulation is not suitable for preparing a sustained release formulation of an insoluble active ingredient due to the small elution rate. In such a case, a formulation, wherein a protecting coat is quickly dissolved at a targeting part of digestive organs to disintegrate and disperse the contents, is desired.

For the above purpose, it is thought suitable to use a granule wherein a micro-dispersing and rapid-disintegrating type of core particle is coated with an enteric polymer controlling the dissolving pH. However, an enteric coated granule prepared through a well known method can not achieve the above purpose. In the conventional formulation, it was often found that the intensity against wetness of the granule quickly became small by a gradual immersion of water, then a burst of the enteric coat granule was caused by a physical stimulation such as an action of digestive organs before reaching the targeting part.

In case of a granule of a soluble active ingredient, even if the disintegration is sacrificed by addition of a gelatinizer or a water repellent to the core granule containing the active ingredient, for keeping the intensity against wetness, the dissolution of the active ingredient can easily be kept good without any problem.

However, in case of a granule of an insoluble active ingredient, the addition of such a gelatinizer or a water repellent agent to the core granule extremely lowers the disintegration, thus effective dissolution can not be expected at lower part of the small intestine or the colon where an amount of water is small.

An enteric coat granule is desired to quickly dissolve the enteric coat at the targeting part, intestine, so as to dissolve or disintegrate and disperse the core particle. However, the above-described burst prevents the enteric coat granule from reaching the targeting part and exhibiting the sustained-release effect. Therefore, an enteric coat granule is desired to reach the targeting part without a burst caused by an action of digestive organs. In detail, a formulation is desired, which is capable of taking an insoluble active ingredient to the targeting part in digestive organs with keeping the intensity against wetness and the rapid-disintegation.

### Disclosure of Invention

The present inventors found that an enteric coat granule comprising a water repellent agent can suppress a burst of itself. The present granule can keep the intensity against wetness and the rapid-disintegration, suppress a burst in digestive organs, provide an appropriate release of an active ingredient and enables an insoluble active ingredient to reach the targeting part in digestive organs.

The present invention provides;
(1) an enteric coat granule having an enteric coat outside of a core particle and having an inner layer inside of the enteric coat and/or an outer layer outside of the enteric coat which comprises a water repellent agent in the inner layer and/or the outer layer,
(2) the enteric coat granule according to the above (1) which comprises a water repellent agent in the enteric coat,
(3) the enteric coat granule according to the above (1) or (2) which comprises a water repellent agent in the inner layer and the outer layer,
(4) the enteric coat granule according to any one of the above (1) to (3) which suppresses a burst of itself caused by an action of digestive organs,
(5) an enteric coat granule which comprises a water repellent agent in an enteric coat and suppresses a burst of itself caused by an action of digestive organs,
(6) the enteric coat granule according to any one of the above (1) to (5) wherein an enteric coating agent in the enteric coat is an enteric coating agent dispersing in water,
(7) the enteric coat granule according to the above (6) wherein the enteric coating agent dispersing in water is hydroxypropylmethylcellulose acetate succinate, methacrylic acid co-polymer LD or methacrylic acid co-polymer S,
(8) the enteric coat granule according to any one of the above (1) to (7) wherein the core particle is a micro-dispersing and rapid-disintegrating type of granule,
(9) the enteric coat granule according to the above (1) to (8) wherein the water repellent agent is hydrogenated castor oil, stearyl alcohol, white beeswax or stearic acid,
(10) the enteric coat granule according to any one of claims (1) to (9) wherein an active ingredient in the core particle is insoluble,
(11) the enteric coat granule according to any one of the above (1) to (10) wherein a water repellent activity is strengthened by spraying a suspension containing a water repellent agent and warming the water repellent agent over the melting point after the dryness process, and
(12) a capsule which comprises the enteric coat granule according to any one of the above (1) to (11) and a rapid dissolving granule.

### Brief Description of Drawings

Figure 1 shows an enteric coat granule comprising a core particle, an inner layer, an enteric coat and an outer layer.
Figure 2 shows the relation between an amount of a water repellent agent in an outer layer and the intensity against wetness.
Figure 3 shows the dissolution pattern of an active ingredient contained in the present granule compared with reference 1 (no water repellent agent) and reference 4 (a water repellent agent is in a core particle).
Figure 4 shows the dissolution pattern of an active ingredient contained in the granule wherein a water repellent agent is in the inner layer and the outer layer.

### Best Mode for Carrying Out the Invention

The present invention is explained in detail below.

It is generally thought that the immersion of water to an enteric coat granule wherein an core particle is covered with an enteric coating agent can be suppressed by the following methods; 1) adding a water repellent agent into a core particle, 2) adding a water repellent agent into an enteric coat or 3) adding a water repellent agent into a core particle and an enteric coat.

However, when an active ingredient is insoluble, it has experimentally been found that the above 1) extremely delays the elution and the disintegration while suppressing the burst. On the other hand, a granule prepared by the above 2) is disclosed as a granule containing a water repellent agent for preventing a granule from attaching to each other, preparing a compact coat, strengthening the intensity against wetness or improving the fluidity in the Japanese Patent Publication (Kokoku 1986-13683), the porpose is not for suppressing the burst caused by an action of digestive organs.

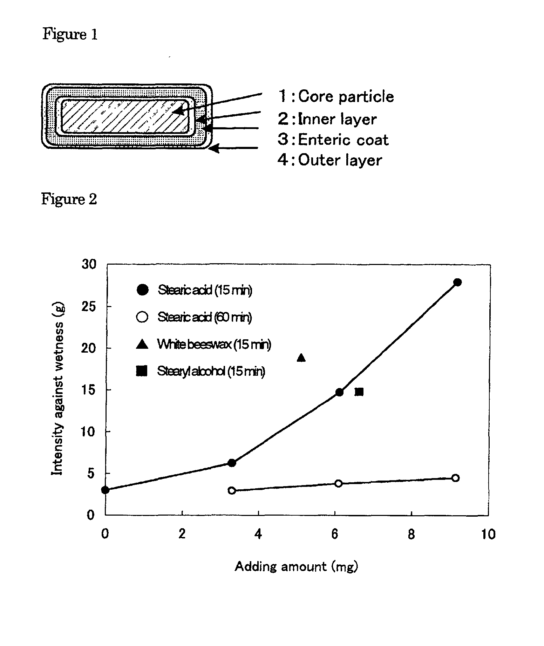

The present inventors have found the immersion of water to in an enteric coat granule wherein a core particle is covered with an enteric coat can be suppressed by the other methods. Such methods include 4) preparing an inner layer inside of the enteric coat, between a core particle and an enteric coat and adding a water repellent agent into the inner layer, 5) preparing an outer layer outside of the enteric coat and adding a water repellent agent into the outer layer and 6) preparing an inner layer and an outer layer and adding a water repellent agent into both the inner layer and the outer layer. Specially, the above 6) is more preferable because the immersion of water can be suppressed at the two layers. Figure 1 shows an enteric coat granule comprising a core particle, an inner layer, an enteric coat and an outer layer.

In the above 4) to 6), the further addition of a water repellent agent into an enteric coat is useful for suppressing a burst of the granule. Specially preferred is a combination with the above 4), a granule prepared by adding a water repellent agent into an inner layer and an enteric coat, and a combination of the above 6), a granule prepared by adding a water repellent agent into an inner layer, an outer layer and an enteric coat.

The present enteric coat granule may include layers other than three layers (e.g., a core particle + an inner layer + an enteric coat, a core particle + an enteric coat + an outer layer) or four layers (e.g., a core particle + an inner layer + an enteric coat + an outer layer) and may form a multi layer granule. When an enteric coat granule includes any layers, at least one of an outer layer or an inner layer comprising a water repellent agent. The other layer may contain a water repellent agent or not. The present enteric coat granule may comprise two or more layers comprising a water repellent agent.

A core particle means a core part of an enteric coat granule comprising an active ingredient and may contain a fine powder of D-mannitol, HPC-SL, L-HPC 31 or the like besides an active ingredient.

An enteric coat means a layer located outside of a core particle, comprising an enteric coating agent and may contain triethyl citrate, a fine powder of talc, sodium lauryl sulfate or the like besides an enteric coating agent.

An enteric coating agent includes an enteric coating agent dissolving in an organic solvent and an enteric coating agent dispersing in water.

Examples of an enteric coating agent dissolving in an organic solvent include methacrylic acid copolymer L (Eudradgit L100), methacrylic acid copolymer LD (Eudradgit L100-559, methacrylic acid copolymer S (Eudradgit S100), hydroxypropylmethylcellulose phthalate 200731 (HPMCP), hydroxypropylmethylcellulose phthalate 220824 (HPMCP), carboxymethylethylcellulose (CMEC) or the like.

Examples of an enteric coating agent dispersing in water include hydroxypropylmethylcellulose acetate succinate (HPMCAS: Shinetsu AQOAT), methacrylic acid copolymer LD (Eudradgit L30D-55), methacrylic acid copolymer S (Eudradgit FS30D) or the like. HPMCAS can be used by neutralizing it with ammonia and dissolving, instead of adding a plasticizer such as triethyl citrate.

A preferable amount of an enteric coating agent is 10 to 70 % by weight, specially 25 to 50 % by weight to that of a core particle,

An inner layer means any layer inside of the enteric coat, any layer between a core particle and an enteric coat. Therefore, as far as an inner layer is between a core particle and an enteric coat, the inner layer does not need to be adjacent to a core particle or an enteric coat. Preferred as an inner layer comprising a water repellent agent is an inner layer adjacent to an enteric coat.

An outer layer means any layer outside of the enteric coat. An outer layer comprising a water repellent agent can exhibit the same effect both when such layer is adjacent to the enteric coat or when such layer is the most outer layer. When a water repellent agent is in the most outer layer, a blocking of each granule may occur under the long term storage. A water repellent agent can be added as a mixture with the other excipient for suppressing the above blocking. Preferred as an outer layer comprising a water repellent layer is an outer layer adjacent to an enteric coat, the most outer layer, and an outer layer just inside of the most outer layer.

An inner layer and an outer layer may include a fine powder of talc or HPMC2910 RW.

A water repellent agent is used for suppressing the immersion of water into a core particle.

Examples of a water repellent agent include fatty oils (e.g., olive oil, orange oil, cacao butter, carnauba wax, tallow, hydrogenated oil, sesame oil, soybean oil, unsaponifiable matter of soybean oil, sunflower oil, camellia oil, corn oil, pig oil, paraffin, light liquid paraffin, bitter chocolate, castor oil, sunflower oil, white beeswax, white petrolatum, rape oil, coconut oil, eucalyptus oil, peanut oil, wheat germ oil, microcrystalline wax, beeswax), fatty acids and their derivatives (e.g., stearic acid, medium chain fatty acid triglyceride, tricaprilin), polyhydric alcohls (e.g., stearyl alcohol, cetanol, myristyl alchol), surfactants (e.g., sucrose fatty acid ester, glyceryl monostearate), perfume (e.g., fennel oil, cinnamon oil, clove oil, turpentine, orange peel oil, peppermint oil), natural high polymers (e.g., purified shellac, shellac, white shellac), the others (tocopherol, silicone resin) or the like.

When a water repellent agent is a solid at room temperature, preferred is a water repellent agent, the melting point of which is 50 to 100 °C. For example, preferred is hydrogenated castor oil, stearyl alcohol, white beeswax or stearic acid. A water repellent agent which is a liquid at room temperature can also be used.

A preferable amount of a water repellent agent is 0.2 to 12 %, especially 2 to 6 % to the total amount of a granule.

The present enteric coat granule comprising a water repellent agent can suppress a burst of itself caused by an action of digestive organs or the like. The burst includes a burst of a granule caused by an action of digestive organs before reaching the targeting part under the condition that an enteric coat and a core particle of an enteric coat granule get soft and the intensity against wetness of the granule decreases, after administering, by the immersion of water existing in digestive organs to an enteric coat granule. Therefore, the present granule can reach a targeting part and release an active ingredient at the targeting time by suppressing a burst.

An active ingredient can be absorbed at the targeting part by selecting an enteric coating agent to be used for an enteric coat granule. Generally, pH in human digestive organs is pH 1 to 5 at stomach, pH 6 at upper small intestine, pH 7 to 8 at lower small intestine and pH 6 to 7 at colon. An enteric coating agent having a different pH for starting the dissolution needs to be selected for controlling a starting time of the drug-release by utilizing the difference of pH in human digestive organs. When a granule is administered to a human after eating a general diet, an average time required for passing digestive organs (a time for releasing 50 % of an active ingredient) is approximately 3 hours at stomach, approximately 4 hours at upper small intestine, approximately 6 hours at lower small intestine and approximately 7 or more hours at colon. Colon is preferable as a targeting part for mostly delaying the drug-releasing time. However, when an active ingredient is insoluble, it is difficult to keep the high absorbability at colon, because the amount of water is small there.

When colon is a targeting part, an enteric coating agent is selected from those dissolving at pH 7 or more, considering the time lag for the dissolution of an enteric coat. For example, when an enteric coating agent dissolving in an organic solvent is used, preferred is methacrylic acid copolymer S (Eudradgit S) or the like. When an enteric coating agent dispersing in water is used, preferred is methacrylic acid copolymer S (Eudradgit FS30S) or the like.

When lower small intestine is a targeting part, an enteric coating agent is selected from those dissolving at pH 6 or 6.5 or more, considering the time lag for the dissolution of an enteric coat. For example, when an enteric coating agent dissolving in an organic solvent is used, preferred is methacrylic acid copolymer L (Eudradgit L100) or the like. When an enteric coating agent dispersing in water is used, hydroxypropylmethylcellulose acetate succinate (Shinetsu AQOAT: HPMCAS-HF) or the like.

When upper small intestine is a targeting part, an enteric coating agent is selected from those dissolving at pH 5 or 5.5 or more, considering the time lag for the dissolution of an enteric coat. For example, when an enteric coating agent dissolving in an organic solvent is used, preferred is methacrylic acid copolymer LD (Eudradgit L100-55), hydroxypropylmethylcellulose phthalate 220824 (HPMCP: Shinetsu HP-50), hydroxypropylmethylcellulose phthalate 200731 (HPMCP: Shinetsu HP-55), carboxy methyl ethyl cellulose (CMEC: Freund OS) or the like. When an enteric coating agent dispersing in water is used, preferred is methacrylic acid copolymer LD (Eudradgit L30D-55), hydroxypropylmethylcellulose acetate succinate (Shinetsu AQOAT: HPMCAS-LF) or the like.

When an enteric coating agent, specially hydroxypropylmethylcellulose acetate succinate is used in an enteric coat, preventing the immersion of water to an enteric coat is very important and the present granule can effectively suppress the immersion of water. The permeability of water (water vapor) to a water dispersion type of membrane formed by an enteric coating agent dispersing in water is 1.2 or several times larger than that of water vapor to a dissolution type of membrane formed by an enteric coating agent dissolving in an organic solvent. The permeability of water vapor to a water dispersion type of membrane formed by an enteric coating agent dispersing in water depends on the size of a dispersion particle used in an enteric coating agent or the dissolubility to a plasticizer. For example, the permeability of water vapor to a water dispersion type of membrane formed by aminoalkylmethacrylate copolymer E, one of enteric coating agents dispersing in water, is 1.2 times larger than that of water vapor to a general dissolution type of membrane.

When water immerse into a core particle, a granule, wherein a core particle is a micro-dispersing and rapid-disintegrating type of granule, is apt to burst. However the present granule can effectively suppress the immersion of water to a core particle and effectively suppress a burst.

The present invention provides an enteric coat granule which suppresses a burst of itself with a water repellent granule. The sustained-release of an insoluble active ingredient is achieved by further using a micro-dispersing and rapid-disintegrating type of granule as a core particle of the present granule.

An enteric coat granule is desired to quickly dissolve the enteric coat at the targeting part and dissolute an active ingredient. Therefore, when an active ingredient is insoluble, a core particle must be prepared so that a granule may rapidly disintegrate and disperse. When an active ingredient is insoluble, preferred as a core particle is a micro-dispersing and rapid-disintegrating type of granule. However, such a micro-dispersing and rapid-disintegrating type of granule is easily immersed by water, so it is important to suppress the immersion of water. Therefore, the present invention is especially useful when an active ingredient is insoluble and a core particle is a micro-dispersing and rapid-disintegrating type of granule.

A micro-dispersing and rapid-disintegrating type of core particle includes a granule disintegrating and dispersing in water within a few minutes (approximately 0.1 to 2 minutes).

The above micro-dispersing and rapid-disintegrating type of core particle can be prepared under the wet granulation by adding to a core particle a disintegrator to be used for keeping the rapid disintegration (e.g., L-HPC31, Ac-Di-Sol, CMC-Ca) and a wetting agent to be used for keep the micro dispersion (e.g., HPC-SL, HPMC2910, polysorbate 80, sodium lauryl sulfate).

An insoluble active ingredient includes an active ingredient, the solubility of which in water at 37 °C is under 0.1 mg/mL, for example, ibuprofen, ethenzamide, indomethacin, nifedipine, griseofulvin, phenacetin, phenobarbital, tolbutamide, phenytoin, prednisolone, (Z)-7-[(1R,2R,3S,5S)-2-(5-hydroxybenzo[b]thiophen-3-ylcarbonylamino)-10-norpinan-3-yl]-5-heptenoic acid (referred to as Compound A in the present specification) or its salt (e.g., its sodium salt, its potassium salt, its calcium salt) or the like.

The sustained release of an insoluble active ingredient can be achieved by preparing the present enteric coat granule comprising a water repellent agent.

It can be judged by measuring the intensity against wetness of a granule whether or not a burst caused by an action of digestive organs can be suppressed, because it directly relates to the physical intensity against wetness of a granule. The intensity against wetness of a granule is measured after a test granule is immersed in a JP-1 solution (described in the 13th revision of Japanese pharmacopeia ) for 15 minutes or 1 hour. GRANO granule hardness meter (Okada Seikou K.K) can be used for a measure. A granule of the present invention shows a high intensity against wetness compared with a granule comprising no water repellent agent and suppress a burst.

An enteric coat granule wherein a burst is suppressed includes an enteric coat granule wherein the immersion of water into a core particle can be suppressed. Preferred is a granule wherein the intensity against wetness in a JP-1 solution for 15 minutes is 5 g or more, 10 g or more, 20 g or more, and 30 g or more.

Preferred as an enteric coat granule wherein a burst caused by an action of digestive organs is an enteric coat granule wherein an active ingredient is not dissoluted in a test solution in which an enteric coating agent can not be dissolved and is dissoluted in a test solution (e.g., a solution consisting of a JP-2 solution and 0.5 % PS80) in which an enteric coating agent and an active ingredient can be dissolved.

An enteric coat granule includes a granule wherein a core particle is covered with an enteric coating agent and may have an inner layer or a outer layer other than an enteric coat.

A granules includes one of granules wherein all the granules can go through a filter (1700 µm), 5 % or less of all the granules remain on a filter (1700 to 1400 µm) and 15 % or less of all the granules can go through a filter (355 µm).

It is necessary for the preparation of a sustained release preparation to mix a rapid dissolving granule and an enteric coat granule. A sustained release preparation can be prepared by mixing an appropriate amount of a rapid dissolving granule and the present enteric coat granule comprising a water repellent agent. The ratio of rapid dissolving granules and enteric coat granules can be decided considering the speed of the release of an active ingredient from each granule, the speed of the dispersion and the dissolution, the speed of the absorption and the metabolism of an active ingredient. For example, the ratio of rapid dissolving granules and enteric coat granules is 3-4 : 6-7.

A capsule comprising a mixture of these granules can be prepared.

A core particle or the like can be used as a rapid dissolving granule.

For example, the present enteric coat granule comprising a water repellent agent can be prepared in accordance with the following procedure.

A core particle can be prepared in accordance with a known method. For example, it can be prepared through a wet pushing granulation or a rolling granulation.

A water repellent agent can be added as shown below.
A) Spraying a suspension comprising a water repellent agent on a surface of a granule in equipment and warming the obtained granule over the melting point of the water repellent agent on and after the dryness procedure.
B) Spraying a solution of a water repellent agent in a solvent, a melting solution of a water repellent agent or a solution of a water repellent agent on a surface of a granule in equipment to cover with a water repellent agent.
C) Putting a granule and a water repellent agent, or a mixture of a water repellent agent and the other excipient in equipment and warming over the melting point of the water repellent agent to cover a surface of the granule with a water repellent agent.

Procedure described in the above A) to C) can be carried out in a fluidized-bed granulator, a centrifugal fluidized type of coating granulator or an aeration type of coating machine.

When preparing a layer comprising a water repellent agent in the preparation of an enteric coat granule, the procedure described in any one of the above A) to C) can be used. For example, when preparing a granule comprising a water repellent agent in an inner layer, the above procedure can be used for a core particle. When preparing a granule comprising a water repellent agent in an outer layer, the above method can be used to a granule covered with an enteric coating agent. A granule comprising a water repellent agent in an enteric coat can be prepared by carrying out the above procedure at the same time when carrying out the enteric coating. A granule comprising a water repellent agent in any layer desired can be prepared by carrying out the above procedure.

A granule comprising a water repellent agent in a core particle can be prepared by preparing a core particle under a wet granulation with mixing a water repellent agent and warming at the dryness procedure.

Methods of preparing the present enteric coat granule including a water repellent agent are not limited to the above A) to C). Enteric coat granules prepared by the other method are within the scope of the present invention. When a water repellent agent is a liquid at room temperature, the granule can be prepared only by spraying.

When a water repellent agent is a solid at room temperature, the granule can preferably be prepared by warming a water repellent agent over its melting point and adding it as well as the above B) or prepared warming a granule over melting point of a water repellent agent after adding a water repellent agent as well as the above A) or C). In the above A) to C), the effect of water repellent agent increases because a coating with a water repellent agent can be perfect, compared with adding a powder of a water repellent agent.

For the preparation of the present granule, excipients, binding agents, disintegrators, stabilizers, plasticizers, edulcorants, correctives, antioxidants, lubricants, dispersants, fluidizating agents, coloring agents, foaming agents or the like can be used in addition to an active ingredient. For example, lactose, sucrose, D-mannitol, corn starch, calcium hydrogen phosphate, crystalline cellulose, polyvinylpyrrolidone K25 (PVP K25), hydroxypropylcellulose (HPC-SL), hydroxypropylmethylcellulose 2910 (HPMC2910), pregelatinized starch, carmellose, carmellose calcium, carmellose sodium, croscarmellose sodium (Ac-Di-Sol), low substituted hydroxypropylcellulose (L-HPC31), partly pregelatinized starch, sodium carboxymethyl starch, hydrogenated castor oil, fine talc, triethyl citrate, sodium lauryl sulfate or the like can be used.

### Example

Compound A was used as an insoluble active ingredient in the following references and examples. The solubilities of Compound A to several test solutions are shown in Table 1.

**Table 1**

| Test solution | Solubility (µg/mL) | | Final |
|---|---|---|---|
| | 3 hours | 6 hours | pH |
| Water | 5.56 | 5.93 | 5.81 |
| JP-1 Sol. | ND | ND | 1.15 |
| JP-2 Sol. | 81.08 | 81.65 | 6.84 |
| pH4.0 | ND | ND | 4.07 |
| pH6.0 | 15.19 | 15.57 | 6.08 |
| pH7.8 | 487.87 | 489.05 | 7.6 |
| pH8.0 | 652.56 | 675.55 | 7.76 |
| pH8.5 | 6197.64 | 6224.39 | 8.29 |

Enteric coat granules were prepared as shown in the following References in order to compare it with the present enteric coat granule. Reference 1 shows a usual enteric coat granule. Reference 2 shows an enteric coat granule comprising hydrogenated castor oil in a core particle. Reference 3 shows an enteric coat granule comprising gelatinizer in a core particle. Reference 4 shows an enteric coat granule prepared by warming the granule obtained through Reference 2 over the melting point of hydrogenated castor oil.

### Reference 1

### a) Preparation of a core particle

Compound A (125 g), D-mannitol (55 g) and L-HPC31 (10 g) were mixed in LFS-GS-2J type of high speed mixer and further granulated with a granulation solution of 13.0 % HPC-SL (76.92 g). The granulation product obtained by carrying out the above procedure twice was treated by DGL1 type of DOOMGRAN granulator and dried at 50 °C for 70 minutes. The obtained dry product was micronized by P-3 type of power mill. A granule the size of which was over 1000 µm or under 710 µm was removed to prepare a core particle.

### b) Inner coating

The core particle (320 g) prepared through the above a) was coated with a coating solution consisting of the following contents through the usual spray coating procedure in UNIGLATT fluidized-bed granulator to prepare a coated granule the total weight of which was 349.8 g.

| | |
|---|---|
| HPMC2910RW | 30.0g |
| Talc | 70.0g |
| Purified water | 900.0g |
| Total | 1000.0g |

### c) Enteric coating

The coated granule (320 g) prepared through the above b) was coated with a coating solution consisting of the following contents through the usual spray coating procedure in UNIGLATT fluidized-bed granulator to prepare an enteric coat granule the total weight of which was 465.3 g.

| | |
|---|---|
| HPMCAS-LF | 120.0g |
| Triethyl citrate | 24.0g |
| Talc | 36.0g |
| Sodium lauryl sulfate | 3.6g |
| Purified water | 1016.4g |
| Total | 1200.0g |

### Reference 2

### a) Preparation of a core particle

Compound A (125 g), D-mannitol (42.5 g), L-HPC31 (10 g) and hydrogenated castor oil (12.5 g) were mixed in LFS-GS-2J type of high speed mixer and further granulated with a granulation solution of 12.6 % HPC-SL (79.42 g). The granulation product obtained by carrying out the above procedure twice was treated by DGL1 type of DOOMGRAN granulator and dried at 50 °C for 70 minutes. The obtained dry product was micronized by P-3 type of power mill. A granule the size of which was over 1000 µm or under 710 µm was removed to prepare a core particle.

### b) Inner coating

The core particle (320 g) prepared through the above a) was coated with a coating solution consisting of the following contents through the usual spray coating procedure in UNIGLATT fluidized-bed granulator to prepare a coated granule the total weight of which was 350.4 g.

| | |
|---|---|
| HPMC2910RW | 30.0g |
| Talc | 70.0g |
| Purified water | 900.0g |
| Total | 1000.0g |

### c) Enteric coating

The coated granule (320 g) prepared through the above b) was coated with a coating solution consisting of the following contents through the usual spray coating procedure in UNIGLATT fluidized-bed granulator to prepare an enteric coat granule the total weight of which was 462.4 g.

| | |
|---|---|
| HPMCAS-LF | 120.0g |
| Triethyl citrate | 24.0g |
| Talc | 36.0g |
| Sodium lauryl sulfate | 3.6g |
| Purified water | 1016.4g |
| Total | 1200.0g |

### Reference 3

### a) Preparation of a core particle

Compound A (125 g), D-mannitol (54.375 g), L-HPC31 (10 g) and L-HPC30 (2.5 g) were mixed in LFS-GS-2J type of high speed mixer and further granulated with a granulation solution of 12.3 % HPC-SL (81.25 g). The granulation product obtained by carrying out the above procedure twice was treated by DGL1 type of DOOMGRAN granulator and dried at 50 °C for 70 minutes. The obtained dry product was micronized by P-3 type of power mill. A granule the size of which was over 1000 µm or under 710 µm was removed to prepare a core particle.

### b) Inner coating

The core particle (320 g) prepared through the above a) was coated with a coating solution consisting of the following contents through the usual spray coating procedure in UNIGLATT fluidized-bed granulator to prepare a coated granule the total weight of which was 354.6 g.

| | |
|---|---|
| HPMC2910RW | 30.0g |
| Talc | 70.0g |
| Purified water | 900.0g |
| Total | 1000.0g |

### c) Enteric coating

The coated granule (320 g) prepared through the above b) was coated with a coating solution consisting of the following contents through the usual spray coating procedure in UNIGLATT fluidized-bed granulator to prepare an enteric coat granule the total weight of which was 461.4 g.

| | |
|---|---|
| HPMCAS-LF | 120.0g |
| Triethyl citrate | 24.0g |
| Talc | 36.0g |
| Sodium lauryl sulfate | 3.6g |
| Purified water | 1016.4g |
| Total | 1200.0g |

### Reference 4

The enteric coat granule prepared through Reference 2 was treated with a heat of 90 °C for 90 minutes in a temperature chamber.

The enteric coat granules of the present invention are shown below.
Examples 1 and 2: Granules comprising a water repellent agent in an inner layer.
Examples 3 and 4: Granules comprising a water repellent agent in an enteric coat.
Example 5: A Granule comprising a water repellent agent in an inner layer and an enteric coat.
Examples 6 to 10: Granules comprising a water repellent agent in an outer layer.
Examples 11 and 12: Granules comprising a water repellent agent in an inner layer and an outer layer.

### Example 1

### a) Preparation of a core particle

Compound A (1000 g), D-mannitol (440.0 g) and L-HPC31 (80 g) were mixed in FS-GS-10J type of high-speed mixer and further granulated with a granulation solution of 13.0 % HPC-SL (615.4 g). The granulation product obtained by carrying out the above procedure twice was treated by DGL1 type of DOOMGRAN granulator and dried at 50 °C for 70 minutes. The obtained dry product was micronized by P-3 type of power mill. A granule the size of which was over 1000 µm or under 710 µm was removed to prepare a core particle.

### b) Inner coating

The core particle (320 g) obtained in the above a) was coated with a coating solution consisting of the following contents through the usual spray coating procedure in UNIGLATT fluidized-bed granulator to prepare a coated granule the total weight of which was 351.6 g. The obtained granule was treated with a heat of 80 °C for 30 minutes to prepare a coated granule.

| | |
|---|---|
| HPMC2910RW | 30.0 g |
| Talc | 64.0 g |
| Stearyl alcohol | 6.0 g |
| Purified water | 900.0 g |
| Total | 1000.0 g |

### c) Enteric coating

The coated granule (320 g) obtained in the above b) was coated with a coating solution consisting of the following contents through the usual spray coating procedure in UNIGLATT fluidized-bed granulator to prepare an enteric coat granule the total weight of which was 462.8 g.

| | |
|---|---|
| HPMCAS-LF | 120.0g |
| Triethyl citrate | 24.0g |
| Talc | 36.0g |
| Sodium lauryl sulfate | 3.6g |
| Purified water | 1016.4g |
| Total | 1200.0g |

### Example 2

### a) Inner coating

The core particle (320 g) obtained in the above a) of Example 1 was coated with a coating solution consisting of the following contents through the usual spray coating procedure in UNIGLATT fluidized-bed granulator to prepare a coated granule the total weight of which was 352.0 g. The obtained granule was treated with a heat of 80 °C for 30 minutes to prepare a coated granule.

| | |
|---|---|
| HPMC2910RW | 30.0g |
| Talc | 58.0g |
| Stearyl alcohol | 12.0g |
| Purified water | 900.0g |
| Total | 1000.0g |

### b) Enteric coating

The coated granule (320 g) obtained in the above a) was coated with a coating solution consisting of the following contents through the usual spray coating procedure in UNIGLATT fluidized-bed granulator to prepare an enteric coat granule the total weight of which was 456.9 g.

| | |
|---|---|
| HPMCAS-LF | 120.0g |
| Triethyl citrate | 24.0g |
| Talc | 36.0g |
| Sodium lauryl sulfate | 3.6g |
| Purified Water | 1016.4g |
| Total | 1200.0g |

### Example 3

### a) Inner coating

The core particle (320 g) obtained in the above a) of Example 1 was coated with a coating solution consisting of the following contents through the usual spray coating procedure in UNIGLATT fluidized-bed granulator to prepare a coated granule the total weight of which was 350.0 g.

| | |
|---|---|
| HPMC2910RW | 30.0g |
| Talc | 70.0g |
| Purified Water | 900.0g |
| Total | 1000.0g |

### b) Enteric coating

The coated granule (320 g) obtained in the above a) was coated with a coating solution consisting of the following contents through the usual spray coating procedure in UNIGLATT fluidized-bed granulator to prepare a coated granule the total weight of which was 461.7 g. The obtained granule was treated with a heat of 80 °C for 30 minutes to prepare an enteric coat granule.

| | |
|---|---|
| HPMCAS-LF | 120.0g |
| Triethyl citrate | 24.0g |
| Talc | 36.0g |
| Stearyl alcohol | 12.0g |
| Sodium lauryl sulfate | 3.6g |
| Purified water | 1004.4g |
| Total | 1200.0g |

### Example 4

### a) Inner coating

The core particle (320 g) obtained in a) of the above Example 1 was coated with a coating solution consisting of the following contents through the usual spray coating procedure in UNIGLATT fluidized-bed granulator to prepare a coated granule the total weight of which was 348.2 g.

| | |
|---|---|
| HPMC2910RW | 30.0g |
| Talc | 70.0g |
| Purified water | 900.0g |
| Total | 1000.0g |

### b) Enteric coating

The coated granule (320 g) obtained in the above a) was coated with a coating solution consisting of the following contents through the usual spray coating procedure in UNIGLATT fluidized-bed granulator to prepare an enteric coat granule the total weight of which was 477.1 g. The obtained granule was treated with a heat of 80 °C for 30 minutes to prepare a coated granule.

| | |
|---|---|
| HPMCAS-LF | 120.0g |
| Triethyl citrate | 24.0g |
| Talc | 36.0g |
| Stearyl alcohol | 24.0g |
| Sodium lauryl sulfate | 3.6g |
| Purified water | 992.4g |
| Total | 1200.0g |

### Example 5

### a) Inner coating

The core particle (320 g) obtained in a) of the above Example 1 was coated with a coating solution consisting of the following contents through the usual spray coating procedure in UNIGLATT fluidized-bed granulator to prepare a coated granule the total weight of which was 349.8 g. The obtained granule was treated with a heat of 80 °C for 30 minutes to prepare a coated granule.

| | |
|---|---|
| HPMC2910RW | 30.0g |
| Talc | 58.0g |
| Stearyl alcohol | 12.0g |
| Purified water | 900.0g |
| Total | 1000.0g |

### b) Enteric coating

The coated granule (320 g) obtained in the above a) was coated with a coating solution consisting of the following contents through the usual spray coating procedure in UNIGLATT fluidized-bed granulator to prepare an enteric coat granule the total weight of which was 468.2 g. The obtained granule was treated with a heat of 80 °C for 30 minutes to prepare a coated granule.

| | |
|---|---|
| HPMCAS-LF | 120.0g |
| Triethyl citrate | 24.0g |
| Talc | 36.0g |
| Stearyl alcohol | 12.0g |
| Sodium lauryl sulfate | 3.6g |
| Purified water | 1004.4g |
| Total | 1200.0g |

### Example 6

### a) Outer coating

The enteric core particle (50.86 g) obtained in c) of Reference 1 was coated with stearyl alcohol (2.07 g) in SUS beaker under warming at 85 °C for 3 minutes to prepare a coated granule. The effective attachment rate is 64.0 %.

### Example 7

### a) Outer coating

The enteric core particle (50.86 g) obtained in c) of Reference 1 was coated with white beeswax (1.35 g) in SUS beaker under warming at 90 °C for 3 minutes to prepare a coated granule. The effective attachment rate is 75.0 %.

### Example 8

### a) Outer coating

The enteric core particle (50.86 g) obtained in c) of Reference 1 was coated with stearic acid (0.81 g) in SUS beaker under warming at 95 °C for 3 minutes to prepare a coated granule. The effective attachment rate is 81.3 %.

### Example 9

### a) Outer coating

The enteric core particle (50.86 g) obtained in c) of Reference 1 was coated with stearic acid (1.46 g) in SUS beaker under warming at 95 °C for 3 minutes to prepare a coated granule. The effective attachment rate is 83.3 %.

### Example 10

### a) Outer coating

The enteric core particle (50.86 g) obtained in c) of Reference 1 was coated with stearic acid (1.99 g) in SUS beaker under warming at 95 °C for 3 minutes to prepare a coated granule. The effective attachment rate is 92.0 %.

### Example 11

### a) Outer coating 1

The granule (300 g) obtained in b) of Example 2 was coated with stearyl alcohol (12.35 g) in UNIGLATT fluidized-bed granulator to prepare a coated granule, The effective attachment rate is 85.0 %.

### b) Outer coating 2

The granule (300 g) obtained in the above a) was coated with a coating solution consisting of the following contents through the usual spray coating procedure in UNIGLATT fluidized-bed granulator to prepare a coated granule the total weight of which was 314.1 g.

| | |
|---|---|
| HPMC2910RW | 20.0g |
| Talc | 26.7g |
| Titanium oxide | 53.3g |
| Purified water | 900.0g |
| Total | 1000.0g |

### Example 12

### a) Inner coating

The core particle (320 g) obtained in a) of Example 1 was coated with a coating solution consisting of the following contents through the usual spray coating procedure in UNIGLATT fluidized-bed granulator to prepare a coated granule the total weight of which was 349.5 g. The obtained granule was treated with a heat of 90 °C for 30 minutes to prepare a coated granule.

| | |
|---|---|
| HPMC2910RW | 30.0g |
| Talc | 58.0g |
| Stearic acid | 12.0g |
| Purified water | 900.0g |
| Total | 1000.0g |

### b) Enteric coating

The coated granule (320 g) obtained in the above a) was coated with a coating solution consisting of the following contents through the usual spray coating procedure in UNIGLATT fluidized-bed granulator to prepare a coated granule the total weight of which was 459.7 g.

| | |
|---|---|
| HPMCAS-LF | 120.0g |
| Triethyl citrate | 24.0g |
| Talc | 36.0g |
| Sodium lauryl sulfate | 3.6g |
| Purified water | 1016.4g |
| Total | 1200.0g |

### c) Outer coating 1

The coated granule (300 g) obtained in the above b) was coated with stearic acid (12.36 g) in UNIGLATT fluidized-bed granulator under warming at 95 C for 15 minutes to prepare a coated granule. The effective attachment rate is 86.9 %.

### d) Outer coating 2

The granule (300 g) obtained in the above c) was coated with a coating solution consisting of the following contents through the usual spray coating procedure in UNIGLATT fluidized-bed granulator to prepare a coated granule the total weight of which was 318.5 g.

| | |
|---|---|
| HPMC2910RW | 20.0g |
| Talc | 26.7g |
| Titanium oxide | 53.3g |
| Purified water | 900.0g |
| Total | 1000.0g |

The contents and the data of the intensity against wetness of the enteric coat granules prepared through the above References and Examples are shown below.

The intensity against wetness was measured in accordance with the following manner. A load cell (50 g) and flat type of tip (φ 0.5 mm) were set on GRANO granule hardness meter (Okada Seikou K.K). A test granule was put on a test stage after the test granule was immersed in a JP-1 solution for 15 or 60 minutes. The maximum loading value necessary to break a granule was measured at a speed of 100 µm/sec and used as the intensity against wetness.

**Table 2**

| Lot No. Different point | | Reference 1 Reference | Reference 2 Hydrogenated castor oil | Reference 3 Gelatinizer |
|---|---|---|---|---|
| Core particle | Compound A | 100 | 100 | 100 |
| | D-mannitol | 44 | 34 | 43.5 |
| | L-HPC 31 | 8 | 8 | 8 |
| | Hydrogenated castor oil | - | 10 | - |
| | L-HPC 30 | - | - | 2 |
| | HPC-SL | 8 | 8 | 8 |
| | (Total) | (160) | (160) | (160) |
| Inner layer | Talc | 10.43 | 10.43 | 12.11 |
| | HPMC2910 RW | 4.47 | 4.47 | 5.19 |
| | (Total) | (14.9) | (15.2) | (17.3) |
| Enteric coat | HPMCAS-LF | 51.90 | 50.96 | 51.22 |
| | Trityl citrate | 10.38 | 10.19 | 10.24 |
| | Talc | 15.57 | 15.29 | 15.37 |
| | Sodium lauryl sulfate | 1.56 | 1.53 | 1.54 |
| | (Total) | (79.40) | (77.96) | (78.37) |
| | Total | 254.30 | 253.16 | 255.67 |
| Intensity against wetness (g) | JP-1 0.25Hr | 3.0 | 4.0 | 6.6 |
| | JP-1 1.00Hr | ND | ND | 2.0 |

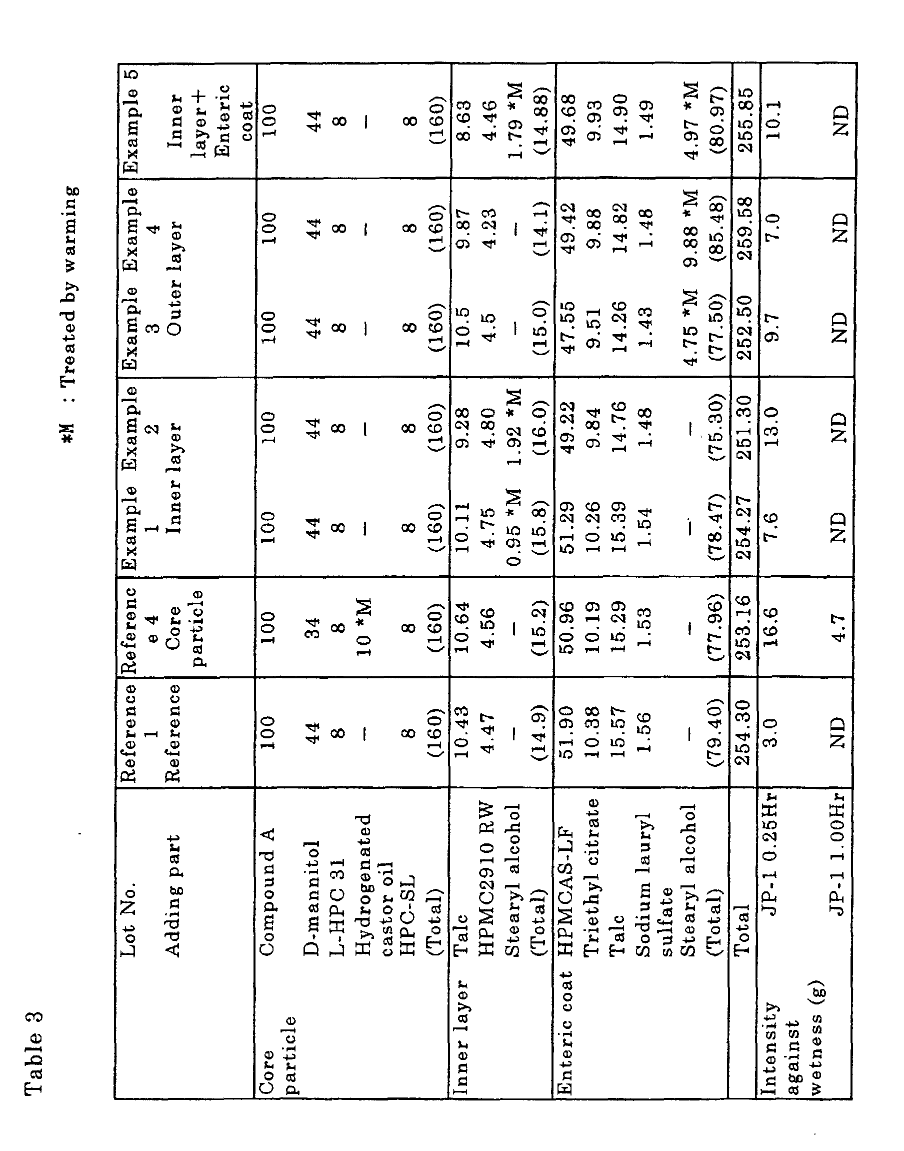

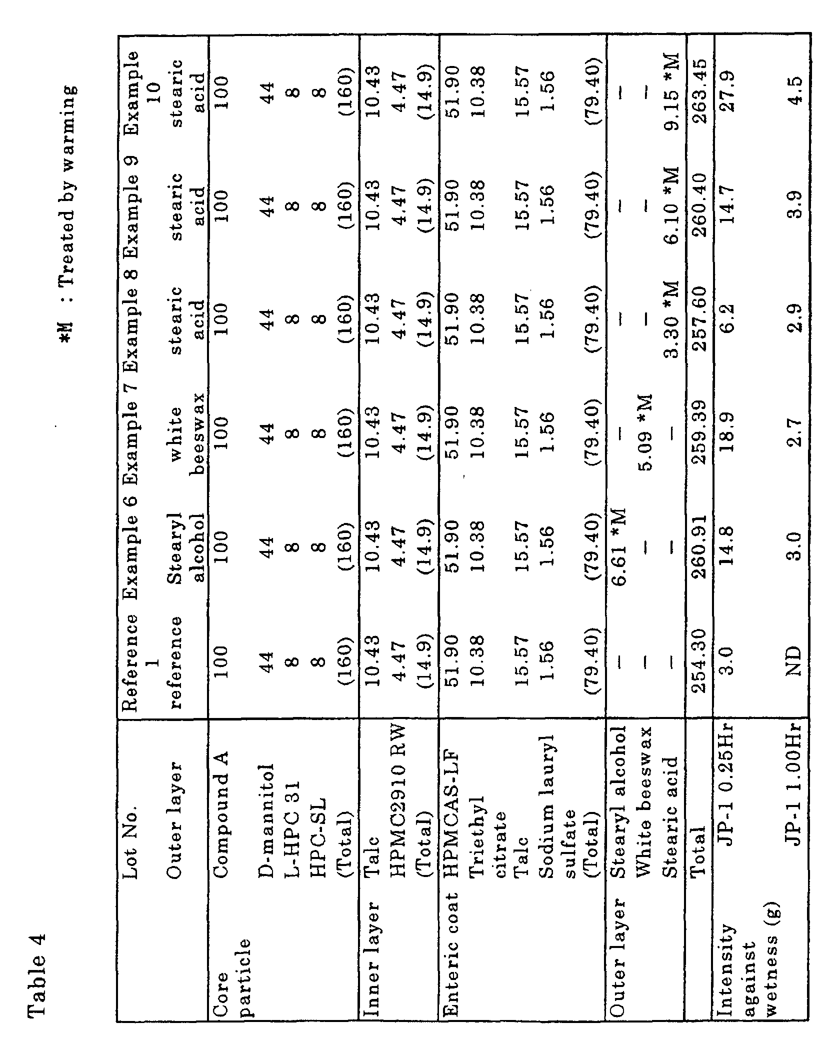

**Table 5**

| Lot No. Water repellent agent Adding part | | Example 11 stearyl alcohol Inner and Outer | Example 12 stearic acid Inner and outer |
|---|---|---|---|
| Core particle | Compound A | 100 | 100 |
| | D-mannitol | 44 | 44 |
| | L-HPC 31 | 8 | 8 |
| | HPC-SL | 8 | 8 |
| | (Total) | (160) | (160) |
| Inner layer | Fine talc | 9.28 | 8.54 |
| | HPMC2910 RW | 4.80 | 4.42 |
| | Stearyl alcohol | 1.92 *M | - |
| | stearic acid | - | 1.77 *M |
| | (Total) | (16.0) | (14.73) |
| Enteric coat | HPMCAS-LF | 49.22 | 49.85 |
| | Triethyl citrate | 9.84 | 9.97 |
| | Talc | 14.76 | 14.95 |
| | Sodium lauryl | 1.48 | 1.50 |
| | sulfate | | |
| | (Total) | (75.30) | (76.27) |
| Outer layer 1 | Stearyl alcohol | 8.80 *M | - |
| | Stearic acid | - | 8.99 *M |
| Outer layer 2 | Titanium oxide | 6.52 | 8.55 |
| | A-HR | | |
| | Talc | 3.26 | 4.28 |
| | HPMC2910 RW | 2.44 | 3.21 |
| | (Total) | (12.22) | (16.04) |
| | Total | 272.32 | 276.03 |
| Intensity | JP-1 0.25Hr | 36.7 | 32.0 |
| | against | | |
| | wetness (g) | | |
| | JP-1 1.00Hr | 6.0 | 6.5 |

| | | | |
|---|---|---|---|
| *M : Treated by warming | | | |

The above Tables show the intensity against wetness of an enteric coat granule is improved by adding a water repellent agent. The intensity against wetness of granules comprising a water repellent agent in an outer layer and an inner layer (Reference 11 and 12) after the immersion in a JP-1 solution for 15 minutes is over 30 g. The present granule can effectively suppress a burst of itself.

The relation between an amount of a water repellent agent in an outer layer and the intensity against wetness in a JP-1 solution for 15 or 60 minutes is shown in Figure 2. Figure 2 shows the intensity against wetness increases by increasing an amount of a water repellent agent to be used. This results shows the addition of a water repellent agent is effective for suppressing a burst.

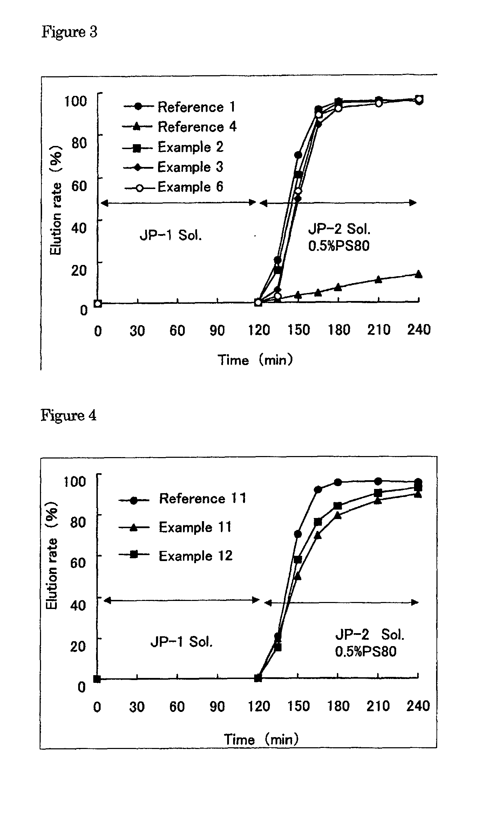

The dissolution of an active ingredient was tested in some of the above enteric coat granules of the present invention. The results are shown in Figure 3 and 4. Figure 3 shows the dissolution of an active ingredient in a test solution consisting of a JP-2 solution and 0.5% PS80 extremely decreases in a granule comprising a water repellent agent in a core particle (Reference 4).

On the other hand, granules comprising a water repellent agent in an inner layer, an enteric coat or an outer layer (Reference 2, 3 or 6) and granules comprising a water repellent agent in an inner layer and an outer layer (Reference 11 and 12) exhibit the good dissolution of an active ingredient in a test solution consisting of a JP-2 solution and 0.5% PS80.

### Industrial Applicability

An enteric coat granule comprising an appropriate amount of a water repellent agent can suppress a burst of itself caused by an action of digestive organs without decreasing the dissolution of an active ingredient.

## Claims

1. An enteric coat granule having an enteric coat outside of a core particle and having an inner layer inside of the enteric coat and/or an outer layer outside of the enteric coat which comprises a water repellent agent in the inner layer and/or the outer layer.

2. The enteric coat granule according to claim 1 which comprises a water repellent agent in the enteric coat.

3. The enteric coat granule according to claim 1 or 2 which comprises a water repellent agent in the inner layer and the outer layer.

4. The enteric coat according to any one of claims 1 to 3 where suppresses a burst of itself caused by an action of digestive organs.

5. An enteric coat granule which comprises a water repellent agent in an enteric coat and suppresses a burst of itself caused by an action of digestive organs.

6. The enteric coat granule according to any one of claims 1 to 5 wherein an enteric coating agent in the enteric coat is an enteric coating agent dispersing in water.

7. The enteric coat granule according to claim 6 wherein the enteric coating agent dispersing in water is hydroxypropylmethylcellulose acetate succinate, methacrylic acid co-polymer LD or methacrylic acid co-polymer S.

8. The enteric coat granule according to any one of claims 1 to 7 wherein the core particle is a micro-dispersing and rapid-disintegrating type of granule.

9. The enteric coat granule according to claim 1 to 8 wherein the water repellent agent is hydrogenated castor oil, stearyl alcohol, white beeswax or stearic acid.

10. The enteric coat granule according to any one of claims 1 to 9 wherein an active ingredient in the core particle is insoluble.

11. The enteric coat granule according to any one of claims 1 to 10 wherein a water repellent activity is strengthened by spraying a suspension containing a water repellent agent and warming the water repellent agent over the melting point after the dryness process.

12. A capsule which comprises the enteric coat granule according to any one of claims 1 to 11 and a rapid dissolving granule.
